# EUROPÄISCHE PATENTSCHRIFT

(11) **EP 1 161 963 B1**
(45) Veröffentlichungstag und Bekanntmachung des Hinweises auf die Patenterteilung: **19.01.2005**
(21) Anmeldenummer: 01113714.8
(22) Anmeldetag: 05.06.2001
(51) Int. Cl.: A61M 16/00, A61H 31/00, A62B 7/00, A62B 9/00, A61M 15/00, A61M 11/00, A61M 16/12, A61M 16/20

(54) **Beatmungsgerät**
Respirator
Respirateur

(30) Priorität: 06.06.2000 DE 20010142 U; 13.10.2000 DE 10050774
(43) Veröffentlichungstag der Anmeldung: 12.12.2001
(73) Patentinhaber: Bösherz, Jakob, 94116 Hutthurm (DE); Götz, Alois H., Dr., 91257 Pegnitz (DE)
(72) Erfinder: Bösherz, Jakob, 94116 Hutthurm (DE); Götz, Alois H., Dr., 91257 Pegnitz (DE)
(74) Vertreter: Blaumeier, Jörg - Patentanwalt

(56) Entgegenhaltungen:
- EP-A- 0 756 502
- EP-A- 0 990 448
- WO-A-00/20061
- US-A- 5 538 002

## Beschreibung

Die Erfindung betrifft ein Beatmungsgerät, mit einem mit einem Patienten verbindbaren Flusseinsteller für in- und exspiratorisches Gas, der ein zur Umgebung hin offenes distales und ein dem Patienten zugewandtes proximales Ende aufweist, und in dessen Innerem eine Düseneinrichtung vorgesehen ist, der abhängig vom jeweiligen Atemzyklus aus wenigstens einer externen Gasquelle ein Gas oder ein Gasgemisch zuführbar ist, wodurch vor und hinter der Düseneinrichtung unterschiedliche Druckverhältnisse erzeugbar sind, und der eine Blendeneinrichtung zugeordnet ist, und mit einer die Gas- oder Gasgemischzufuhr und die Funktion der Blendeneinrichtung steuernden Steuerungseinrichtung.

Ein derartiges Beatmungsgerät ist beispielsweise aus dem europäischen Patent EP 0 756 502 B1 bekannt. Dort ist ein relativ langer rohrartiger Beatmungskanal vorgesehen, an dem geeignete Hochdrucksgasanschlüsse für Beatmungsgase vorgesehen sind. Der Beatmungskanal ist in Form eines Venturi-Rohrs mit einer Variodüse mit einstellbarem Querschnitt ausgestattet. Diese Variodüse ist flussabwärts einer Injektordüse zugeordnet. Ferner ist eine Varioblende mit einstellbarem Querschnitt flussaufwärts der Injektordüse vorgesehen, welche die Menge der aufgrund des Venturi-Effektes einsaugbaren Raumluft dosiert begrenzt. Die Beatmungsparameter, also die Gaszufuhr, Gasmengenregelung sowie die Einstellung der Varioblende erfolgt über eine zentrale Steuerungseinrichtung. Bei diesem Beatmungsgerät ist am Beatmungskanal ein endexspiratorischer Sauerstoffkonzentrationssensor vorgesehen, dessen Ausgangssignale zur Regelung des variablen Querschnitts der Varioblende und zur Einstellung des Querschnitts der Variodüse verwendet wird.

Dieses Beatmungsgerät unter Verwendung des in oben beschriebener Weise ausgebildeten Beatmungskanals ist jedoch in mehrererlei Hinsicht nachteilig. Ein besonders gravierender Nachteil liegt darin, dass insbesondere die variable Blende nur sehr schwer hergestellt werden kann. Das Problem liegt darin, dass der innere Rand der Blende während der Verengung die konzentrische Form verliert, was die Druckleistung des als Flusseinsteller oder Flowgenerator benennbaren Beatmungskanals verschlechtert. Ein weiteres Problem besteht darin, dass die Blende zwischen einem beachtlich großen maximalen Innendurchmesser und einem sehr kleinen minimalen Innendurchmesser variierbar sein muß, um zum einen je nach Beatmungsart unterschiedliche Gasmengen bzw. Mengen an Raumluft ansaugen zu können. So ist es im Rahmen einer normalen Beatmung eines Erwachsenen beispielsweise erforderlich, pro Atemzyklus über die Düseneinrichtung 700 ml bis 800 ml zuzuführen, während bei einem Kleinkind wesentlich weniger Luft, z.B. nur 50 - 100 ml zugeführt werden müssen. Ferner muß bei Exspiration die Düseneinrichtung vollständig geöffnet sein, um die Ausatemluft aus dem offenen System abführen zu können. Insgesamt ist es bei dem vorbekannten Beatmungsgerät erforderlich, die Blendeneinrichtung so auszugestalten, dass sie zwischen einem maximalen Innendurchmesser von ca. 10 mm auf einen minimalen Durchmesser von ca. 2 mm verstellt werden kann, was aus fertigungstechnischer Sicht äußerst schwierig ist. Entsprechendes gilt natürlich für die im Durchmesser ebenfalls veränderbare Variodüse.

Der Erfindung liegt damit das Problem zugrunde, ein dem gegenüber verbessertes Beatmungsgerät anzugeben.

Zur Lösung dieses Problems ist bei einem Beatmungsgerät der eingangs genannten Art erfindungsgemäß vorgesehen, dass die Düsen- und die Blendeneinrichtung mehrere parallele, verteilt angeordnete Düsen mit jeweils einer Blende zum Variieren des jeweiligen Düsenquerschnitts aufweist, wobei die Anzahl der mit dem Gas oder Gasgemisch beaufschlagbaren Düsen und der zur Querschnittsvariation zu betätigenden Blenden bedarfsabhängig wählbar ist.

Beim erfindungsgemäßen Beatmungsgerät sind an Stelle der lediglich einen Düse mit zugeordneter Blende mehrere verteilte Düsen mit zugeordneten Blenden vorgesehen. Dabei ist die Anzahl der betätigten Düsen und Blenden bedarfsabhängig wählbar, d.h., je nach gewünschtem Beatmungsmuster oder Inspirations- bzw. Exspirationszyklus ist es möglich, unterschiedlich viele Düsen samt Blenden anzusteuern. Auf diese Weise ist es möglich, zum einen unterschiedliche Beatmungsformen bis hin zur Hochfrequenzbeatmung, Hochfrequenzoszillation sowie verschiedene Kombinationen mit herkömmlichen Beatmungsverfahren durch entsprechende Düsen- und Blendensteuerung mit dem erfindungsgemäßen Gerät durchzuführen. Weiterhin kann durch einfache Auswahl der angesteuerten Düsenund Blendenanzahl zwischen einem sich insgesamt ergebenen minimalen Düsenquerschnitt - wenn also nur eine Düse mit ihrer Blende angesteuert wird, die den Düsenquerschnitt sogar noch weiter verengen kann - und einem maximalen Düsenquerschnitt - wenn also alle Düsen und Blenden angesteuert und offen sind - variiert werden. Der Flusseinsteller selbst ist z.B. an einem Intubationstubus dicht ansteckbar, wobei die Querschnittsfläche sämtlicher Düsen der Querschnittsfläche des dem Körpergewicht des Patienten entsprechenden Intubationstubus zumindest gleich, bevorzugt aber auch etwas größer ist.

Eine Blende kann erfindungsgemäß durch Gaszufuhr balgartig aufblasbar sein, wobei der Düsenquerschnitt abhängig vom Aufblasgrad ist. Je nachdem wie weit also eine Blende aufgeblasen wird, verengt sich der Düsenquerschnitt. Es kann zwischen einer vollständigen Offenstellung und einem vollständigem Abschluß der Düse je nach Aufblasgrad jeder beliebige Düsenquerschnitt eingestellt werden.

Nach einer ersten erfindungsgemäßen Ausgestaltung kann jede Düse separat mit dem Gas oder Gasgemisch beaufschlagbar und/oder jede Blende separat betätigbar sein. Alternativ dazu ist es auch möglich, dass zumindest ein Teil der Düsen miteinander gekoppelt und gemeinsam mit dem Gas oder Gasgemisch beaufschlagbar ist und/oder das zumindest ein Teil der Blende miteinander gekoppelt und gemeinsam betätigbar sind. Dabei sollte die Kopplungskonfiguration der Düsen und Blenden derart gewählt sein, dass zwischen einer Düse und Blende und der maximalen Anzahl an Düsen und Blenden je die beliebige Düsen- und Blendenanzahl wählbar ist. Zweckmäßigerweise sind mindestens fünf, insbesondere mindestens sieben Düsen und Blenden vorgesehen. Da die Düsen derart bemessen sein sollten - der Innendurchmesser beträgt z.B. ca. 2 mm -, dass über jede Düse pro Atemzug ca. 150 ml Beatmungsgas zugeführt werden können, ist eine Düseneinrichtung mit mindestens fünf Düsen bereits ausreichend, um das für einen Atemzug eines Erwachsenen erforderliche Gasvolumen von ca. 700 ml zuzuführen. Zweckmäßigerweise sind jedoch mindestens sieben vorgesehen, natürlich besteht auch die Möglichkeit, mehr als sieben z. B. zwölf Düsen vorzusehen.

Je nach gewählter Düsenanzahl sind bei Düsen- und Blendenkoppelung zweckmäßigerweise folgende Konfigurationen zu wählen:
Bei fünf Düsen und Blenden:
   - je eine separate Düse und Blende,
   - zwei Paare mit jeweils zwei gekoppelten Düsen bzw. Blenden;
Bei sieben Düsen und Blenden:
   - je eine separate Düse und Blende,
   - zwei Paare mit jeweils zwei gekoppelten Düsen bzw. Blenden,
   - zwei Paare mit jeweils vier gekoppelten Düsen bzw. Blenden;
Bei zwölf Düsen und Blenden:
   - je eine separate Düse und Blende,
   - zwei Paare mit jeweils zwei gekoppelten Düsen bzw. Blenden,
   - zwei Paare mit jeweils vier gekoppelten Düsen bzw. Blenden,
   - zwei Paare mit jeweils fünf gekoppelten Düsen bzw. Blenden.

Diese Art der Koppelung ermöglicht es, durch wahlweises Zuschalten der einzelnen oder gekoppelten Blenden jede beliebige Düsen- und Blendenzahl zwischen 1 und n anzusteuern und so in optimaler Weise auf die gegebenen Anforderungen reagieren zu können.

Die Düsen und die Blenden können jeweils synchron beaufschlägbar bzw. betätigbar sein. D.h., sämtliche anzusteuernden Düsen werden jeweils gleichzeitig und synchron angesteuert, entsprechendes gilt für die Blenden. Alternativ ist auch eine asynchrone Ansteuerung möglich, d.h., pro Atemzyklus werden die zu betätigenden Düsen und Blenden beispielsweise kurz hintereinander zeitlich versetzt angesteuert. Damit können beliebige Flowmuster realisiert werden.

Zur Düsen- und Blendenbetätigung ist zweckmäßigerweise jeweils eine über die Steuerungseinrichtung steuerbare Verteilereinrichtung zum Zuführen des Gases oder Gasgemischs an die eine oder mehreren Düsen und des Aufblasgases an die eine oder mehreren Blenden vorgesehen. Über diese Verteilereinrichtung wird, je nach Vorgabe seitens der Steuerungseinrichtung das jeweils zuzuführende Gas auf die Düsen/Blenden verteilt. Die Düsen/Blenden sind über entsprechende Leitungen mit der jeweiligen Verteilereinrichtung gekoppelt. Je nach dem, ob jede Düse/Blende separat angesteuert werden kann, oder ob gekoppelte Düsen/Blenden vorliegen sind mehr oder weniger Leitungen vorhanden. Diese Leitungen sind äußerst dünn, der Leitungsinnendurchmesser liegt im Bereich von ca. 1,5 mm. Da die Gase mit hohem Druck zugeführt werden ist es möglich, trotz des äußerst geringen Leitungsdurchmessers eine hinreichende Gasmenge zur Verfügung zu stellen. Dieser sehr geringe Leitungsdurchmesser ist ferner dahingehend von Vorteil, als der Totraum des Systems sehr klein ist.

Zweckmäßig ist es ferner, wenn ein Einstellmittel, insbesondere ein Proportionalventil zum Einstellen der zugeführten Gasmengen vorgesehen ist. Hierüber wird je nach gewählter Beatmungsform und gewünschter Gaszusammensetzung die entsprechende Einstellung vorgenommen, natürlich gesteuert über die zentrale Steuerungseinrichtung. Zweckmäßigerweise ist der den Düsen zugeordneten Verteilereinrichtung wenigstens ein Proportionalventil mit zugeordnetem Flusssensor und Druckstabilisator vorgeschaltet. Neben der bereits beschriebenen Mengeneinstellung dient das Proportionalventil ferner zur Bestimmung der Flussleistung sowie des Flussmusters des Flusseinstellers, die Messung des zu den Düsen zugeführten Gasflusses erfolgt über den Flusssensor. Der den Blenden zugeordneten Verteilereinrichtung ist erfindungsgemäß wenigstens ein Druckerzeuger und ein Druckstabilisator vorgeschaltet.

Zweckmäßigerweise ist eine zentrale und mittig angeordnete Düse samt Blende vorgesehen, die gegenüber den sie ring- oder kastenartig umgebenden Düsen und Blenden erhöht angeordnet ist, wobei sie um mindestens die Blendenhöhe hervorsteht, damit sichergestellt ist, dass die Blende auch problemlos aufgeblasen werden kann. Die Düsen und Blenden sind z.B. in im Wesentlichen kreisförmiger Konfiguration in einem rohrartigem, vorzugsweise kreisförmigen Flusseinstellergehäuse angeordnet. Alternativ können die Düsen und Blenden auch in einer von einer Kreisform abweichenden Konfiguration in dem rohrförmigen Flusseinstellergehäuse angeordnet sein, z.B. in rechteckiger Anordnung. Daneben besteht natürlich die Möglichkeit, bei Verwendung von z.B. zwölf Düsen und Blenden eine zentrale Düse erhöht anzuordnen, eine erste sie umgebende Ringreihe etwas tieferliegend und die äußerste Ringreihe aus Düsen und Blenden nochmals etwas tieferliegend anzuordnen, der Versatz beträgt jeweils mindestens die Blendenhöhe. Insgesamt ergibt sich dann eine abgestufte Konfiguration.

Jede Düse kann rohrartig ausgebildet sein und einen wesentlichen kreisförmigen Querschnitt aufweisen. Dies ist aus fertigungstechnischen Gründen vorteilhaft, da die Düsen zweckmäßigerweise aus Kunststoff gefertigt sind und mit einem runden Querschnitt leichter gezogen werden können. Insgesamt ist die kreis- und rohrförmige Ausbildung des Flusseinstellers sowie sämtliche seiner Komponenten auch aus hygienischen und reinigungstechnischen Gesichtspunkten vorteilhaft, da in diesem Fall keine Ecken und Kanten gegeben sind, in denen sich Sputum und dergleichen ansammeln kann, das nur umständlich entfernt werden kann. Alternativ dazu kann hier die rohrartige ausgebildete Düse auch einen von einer Kreisform abweichenden Querschnitt aufweisen.

Um eine einfache Verengung des Querschnitts mittels der aufblasbaren Blende zu ermöglichen, ist es besonders vorteilhaft, wenn jede Düse aus einem ersten formstabilen Abschnitt und aus einem zweiten elastisch verformbaren Abschnitt besteht, der mittels der Blende zur Querschnittsvariation reversibel zusammendrückbar ist. Wird die Blende aufgeblasen, so drückt sie gegen den elastisch verformbaren Abschnitt der nach innen gedrückt wird, der Querschnitt wird verringert. Dies kann soweit gehen, dass der Abschnitt vollständig zusammengedrückt wird und die Düse insgesamt abgedichtet ist.

Ferner kann an jeder Düse, insbesondere am formstabilen Abschnitt wenigstens ein Injektionskanal vorgesehen sein, über den das Gas oder Gasgemisch in die Düse einbringbar ist. Zweckmäßigerweise sind mehrere derartige Injektionskanäle über dem Umfang der Düse verteilt angeordnet. Wird Gas oder Gasgemisch über diese Injektionskanäle in die Düse eingeblasen so wird eine trichterförmige Turbulenz erzeugt, die dem Venturi-Effekt ähnlich ist. Hierdurch entsteht am zum Patienten zugewandten Ende des Flusseinstellers ein positiver, erhöhter Druck, am entgegengesetzten, zur Umgebung hin offenen Ende des Flusseinstellers bzw. der Düsen-/Blendenanordnung entsteht ein Unterdruck, der zum Einsaugen von Raumluft aus der Umgebung verwendet wird. Die Menge an eingesaugter Raumluft wird wiederum über die Blendenstellung und damit den Düsenquerschnitt gesteuert. Festzuhalten ist, dass jede Düsenart verwendet werden kann, die es ermöglicht, durch Injizieren des Gases Raumluft anzusaugen. Es können dem gemäß auch Düsen verwendet werden, die exakt nach dem Venturi-Prinzip arbeiten.

Um den Effekt zu optimieren sollte der Injektionskanal unter einem spitzen Winkel bezüglich der Düsenwand und zum unteren, zum Patienten hingerichteten Ende münden. Ein Winkel von ca. 60° ist dabei besonders zweckmäßig, wobei die Erfindung jedoch nicht auf diesen Winkel beschränkt ist.

Um bei einer Anordnung mehrerer Injektionskanäle an einer Düse alle Kanäle gleichzeitig mit Gas oder Gasgemisch versorgen zu können ohne für jeden Injektionskanal eine separate Zuleitung vorsehen zu müssen kann ferner vorgesehen sein, dass an jeder Düse ein im Wesentlichen ringförmiger Verteilerkanal vorgesehen ist, über dem einzubringendes Gas oder Gasgemisch zwischen den an der Düse vorgesehenen Injektionskanälen verteilbar ist. Diese Verteilerkanäle sind ferner dahingehend zweckmäßig, als über sie etwaige gekoppelte Düsen miteinandergekoppelt sein können, so dass über eine Zuleitung auch die mehreren gekoppelten Düsen versorgt werden können. Alternativ kann ein Injektionskanal auch ringförmig ausgebildet sein und die Düse umlaufen, in ihn mündet ein Gasanschluss. Ein Verteilerkanal im oben beschriebenen Sinn ist hier nicht unbedingt mehr nötig, kann aber vorgesehen sein.. Die Kopplung kann über die Injektionskanäle direkt erfolgen oder sofern vorgesehen über die Verteilerkanäle.

Das erfindungsgemäße Beatmungsgerät ist als offenes System mit einem zur Umgebung hin offenen inspiratorischen Leitungsschenkel und einem exspiratorischen Leitungsschenkel ausgebildet. Am Flusseinsteller gehen also die beiden Leitungsschenkel ab, beide sind insoweit zur Umgebung hin offen, wobei die jeweiligen Flussrichtungen über geeignete Richtungsventile, die in den mittels geeigneter Schläuche gebildeten Leitungsschenkel angeordneten sind, definiert werden. Zweckmäßig ist es ferner, wenn erfindungsgemäß mindestens ein mit der Steuerungseinrichtung kommunizierendes Sensorelement zum Erfassen wenigstens eines beatmungsrelevanten Messwertes des In- und/oder Exspirationsgases, in dessen Abhängigkeit die Steuerung der Düsen und Blenden erfolgt, vorgesehen ist.

Ein bevorzugtes Verfahren zum Betrieb eines Beatmungsgeräts der vorbeschriebenen Art, geichnet sich dadurch aus, dass mit wenigstens einem mit einer zentralen Steuerungseinrichtung kommunizierenden Sensorelement ein beatmungsrelevanter Messwert des In- und/oder Exspirationsgases erfasst wird, in dessen Abhängigkeit die Zufuhr eines Gases oder Gasgemisches zu einer beliebigen Anzahl an Düsen und die Betätigung einer beliebigen Anzahl an der Variierung des Düsenquerschnitts dienenden Blenden, von denen je eine einer Düse zugeordnet ist, derart gesteuert wird, dass je nach Bedarf verschiedene Flussgeschwindigkeiten bei konstantem Beatmungsdruck oder konstante Flussgeschwindigkeiten bei variierendem Beatmungsdruck oder ein gewünschtes Gasmischungsverhältnis aus zugeführtem Gas oder Gasgemisch und über die Düsen bei Gas- oder Gasgemischzufuhr ansaugbarer Raumluft einstellbar ist. Es erfolgt hier also eine messwertabhängige Steuerung der Düsen- und Blendenbetätigung, die es ermöglicht, beliebige Fluss- und Beatmungsmodi zu realisieren. Für die Praxis bedeutet dies, dass beliebige bekannte Beatmungsverfahren sowie Hochfrequenzbeatmung und Mischformen üblicher Beatmungsmodi mit Hochfrequenz-Jet-Oszillationen mit einem einzigen Gerät darstellbar sind.

Zum Variieren des Düsenquerschnitts wird eine Blende aufgeblasen, wobei der Düsenquerschnitt in Abhängigkeit des Aufblasgrads variiert. Die Zufuhr des Gases oder Gasgemisches zu der oder den Düsen kann synchron oder asynchron erfolgen, entsprechendes gilt für die Blendenbetätigung.

Da das Gerät als offenes System besonders offener Beatmungskreis ausgebildet ist, ist es dem Patienten auch möglich, in jeder Betriebssituation des Gerätes, also auch wenn dieses ausgeschaltet ist, spontan zu atmen. Um in einem solchem Fall gegebenenfalls unterstützend eingreifen zu können ist es besonders zweckmäßig, wenn zur Erfassung eines spontanen Atemzugs mittels wenigstens eines Sensorselements der Lungendruck erfasst wird, wobei bei Erfassung einer spontanen Lungentätigkeit sofort sämtliche Blenden zur zumindest teilweisen Verengung des jeweiligen Düsenquerschnitts aufgeblasen werden, wobei der Lungendruck patientennah am proximalen Ende des Flusseinstellers abgegriffen wird. Da der Lungendruck direkt am Patienten abgegriffen wird, wird eine einsetzende Lungentätigkeit sofort erfasst, d.h., jede auch noch so geringe Lungenbewegung die zu einer Druckänderung führen kann wird aufgenommen. Ist dies der Fall erfolgt eine Verengung des Düsenquerschnitts um bei einsetzender Inspiration für die erforderlichen Druckverhältnisse zu sorgen.

Ferner ist es von Vorteil, wenn der Innenraum des Flusseinsteller vor jeder Inspiration mit Frischgas besprüht wird. Da das gesamte System nur einen äußerst kleinen Totraum besitzt - wie beschrieben werden insgesamt nur sehr dünne Leitungen verwendet, ferner ist der Flusseinsteller insgesamt sehr klein - kann mit einer sehr geringen Menge an Frischgas der Innenraum des Flusseinstellers gespült werden, so das sich dort keinerlei Exspirationsgas mehr befindet.

Ferner kann vorgesehen sein, dass bei spontaner Atmung die Sauerstoffkonzentration des Inspirationsgases anhand des Messwertes eines ersten Flusssensors in einem inspiratorischen, zur Umgebung hin offenen Leitungsschenkel, eines zweiten Flusssensors in einem exspiratorischen, zur Umgebung hin offenen Leitungsschenkel sowie einen dritten, in einer zum Flusseinsteller führenden Sauerstoffleitung angeordneten Flusssensors und einem das Mischungsverhältnis eines Sauerstoff-Luftgemischs, das in einem Sauerstoffmischer aufbereitet wird, angebenden Messwert rechnerisch ermittelt wird, wonach der Sauerstoffkonzentrationswert mit dem mittels einem im exspiratorischen Leitungsschenkel angeordneten Konzentrationssensors erfassten Wert des exspiratorischen Gases verglichen und in Abhängigkeit des Vergleich die Sauerstoffkonzentration im Inspirationsgases angepaßt wird.

Weitere Vorteile, Merkmale Einzelheiten der Erfindung ergeben sich aus dem im Folgenden beschriebenen Ausführungsbeispiels sowie anhand der Zeichnungen:
- Fig. 1: eine schematische Darstellung eines erfindungsgemäßen Beatmungsgeräts,
- Fig. 2: eine schematische Darstellung eines Flusseinstellers,
- Fig. 3: eine Schnittansicht durch den Flusseinsteller nach Fig. 2 in Richtung der Linie III-III,
- Fig. 4: eine Schnittansicht durch den Flusseinsteller nach Fig. 2 in Richtung der Linie IV-IV,
- Fig. 5: eine Prinzipdarstellung einer Düse samt zugeordneter Blende,
- Fig. 6: eine Schnittansicht entlang der Linie VI-VI durch die Düse nach Fig. 5,
- Fig. 7: eine Schnittansicht in Richtung der Linie VII-VII in Fig. 5

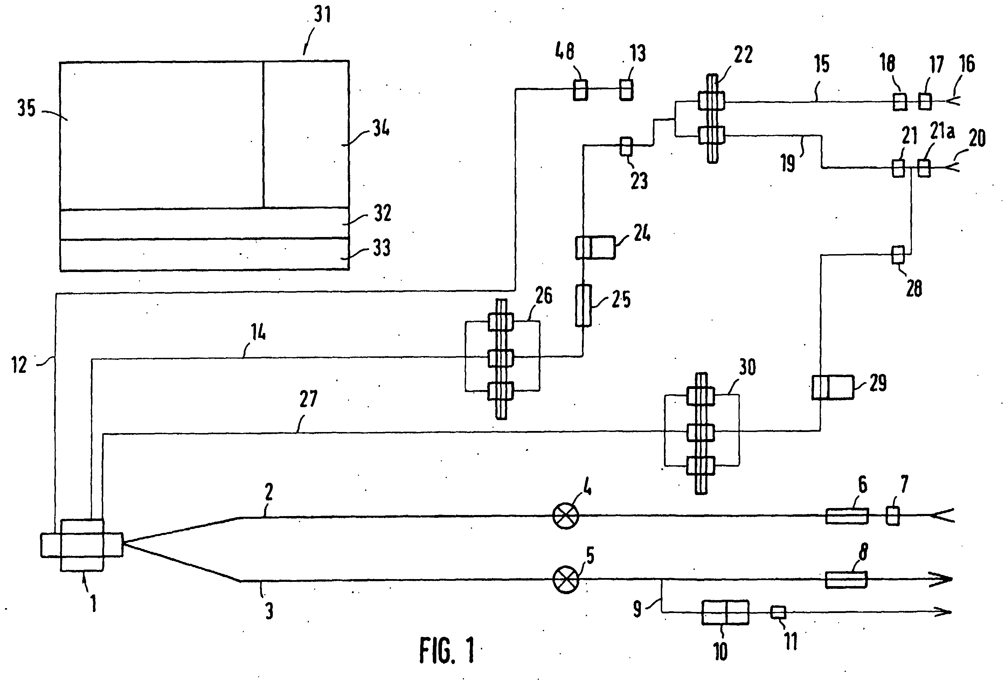

Fig. 1 zeigt ein erfindungsgemäßes Beatmungsgerät umfassend einen Flusseinsteller 1, auf den nachfolgend noch näher eingegangen wird. Am Flusseinsteller 1 münden zwei mittels geeigneter Schläuche gebildete Leitungsschenkel, ein inspiratorische Leitungsschenkel 2 und ein exspiratorischer Leitungsschenkel 3. Über den inspiratorischen Leitungsschenkel 2 kann Raumluft in den Flusseinsteller 1 eingesaugt werden, der exspiratorische Leitungsschenkel 3 dient zur Abfuhr des Exspirationsgases. Die jeweilige Durchlassrichtung wird über geeignete Richtungsventile 4, 5 definiert. Im Leitungsschenkel 2 ist ferner ein inspiratorischer Flusssensor 6 sowie ein Luftfilter 7 vorgesehen, im Leitungsschenkel 3 befindet sich ein exspiratorischer Flusssensor 9. Beide Schenkel 2, 3 sind zur Umgebung hin offen. Am Leitungsschenkel 3 zweigt eine Leitung 9 mit Gassensoren 10 ab, die der Messung der Sauerstoffkonzentration und Kohlendioxidkonzentration im exspiratorischen Gas dienen. Die Gase werden mit einer kontinuierlichen Flussrate mittels der Vakuumpumpe 11 an die Sensoren 10 (mit zugeordneter Messkamera) geleitet.

Über eine Druckleitung 12 ist ein Drucksensor 13 mit vorgeschalteter Wasserfalle 48 mit dem proximalen Ende des Flusseinstellers 1 verbunden. Dieses proximale, dem Patienten zugewandte Ende wird zweckmäßigerweise gasdicht an einem Intubationstubus befestigt. Der Drucksensor 13 dient zum Erfassen des Lungendrucks und damit der Lungentätigkeit. Hierüber kann beispielsweise ein spontaner Atemversuch sofort ermittelt werden, da aufgrund der Ab- oder Zunahme des Drucks direkt am proximalen Ende jede auch noch so geringe Druckschwankung in der Lunge sofort über die extrem dünne Druckleitung 12 an den Drucksensor 13 gegeben werden kann.

Über eine Druckleitung 14 werden dem Patienten Beatmungsgase zugeführt. In Fig. 1 ist lediglich eine Druckleitung 14 dargestellt, insgesamt sind jedoch mehrere Druckleitungen 14 vorgesehen, worauf nachfolgend noch eingegangen wird. In der Druckleitung 14 ist zunächst an einem ersten Leitungsschenkel 15 ein Anschluß 16 für reinen komprimierten Sauerstoff vorgesehen, wobei dem Anschluß 16 ein Gasfilter 17 sowie ein Druckminderer 18 nachgeschalten ist. Im zweiten Schenkel 19 ist ein Eingang 20 für komprimierte Luft vorgesehen, gefolgt von einem Gasfilter 21 a sowie einem Druckminderer 21. Beide Schenkel 15 und 19 münden in einem Sauerstoffmischer 22, der beide Gase gemäß einem gewünschten Mischungsverhältnis mischt. Das Gasgemisch - oder im Falle einer reinen Sauerstoffbeatmung der reine Sauerstoff - wird über einen Druckstabilisator 23 einem Proportionalventil 24 zugeführt, dem wiederum ein Flusssensor 25 sowie eine Verteilereinrichtung 26 nachgeschalten ist. Das Proportionalventil 24 dient zum Modulieren der über die mehreren Druckleitungen 14 den unterschiedlichen Düsen bzw. Düsenkoppelungen zuzuführenden Gase nach Frequenz, Druck und Zeitverhältnissen sowie zum Einstellen der zuzuführenden Gasmengen. Über die Verteilereinrichtung wird die zugeführte Gasmenge auf die jeweiligen Druckleitungen 14 verteilt.

Über eine weitere Druckleitung 27 (auch hier ist in Fig. 1 lediglich eine dargestellt, insgesamt sind jedoch mehrere vorgesehen), die mit dem Schenkel 19 und damit mit dem Eingang 20 für komprimierte Luft verbunden ist, wird den Blenden Druckluft zugeführt, um diese zur Variierung des Düsenquerschnitt aufzublasen. Zu diesem Zweck befindet sich in der Druckleitung 27 ein Druckstabilisator 28 sowie ein Druckerzeuger 29 und eine zweite Verteilereinrichtung 30, die zum Verteilen des zugeführten Gases auf die jeweiligen anzusteuernden Blenden bzw. Blendenkoppelungen dient.

Gesteuert wird der gesamte Betrieb des Beatmungsgeräts bzw. seine jeweiligen Einzelkomponenten mittels einer zentralen Steuerungseinrichtung 31 umfassend eine Rechnereinrichtung 32, eine pneumatische Steuereinheit 33, eine Eingabeeinheit 34 sowie eine Anzeigeeinrichtung vorzugsweise in Form eines Displays oder Monitors 35. Mittels der Steuerungseinrichtung 31 ist es möglich, beliebige Beatmungsmuster einzustellen und ausführen zu lassen, um so mittels eines Geräts optimiert einen Patienten beatmen zu können.

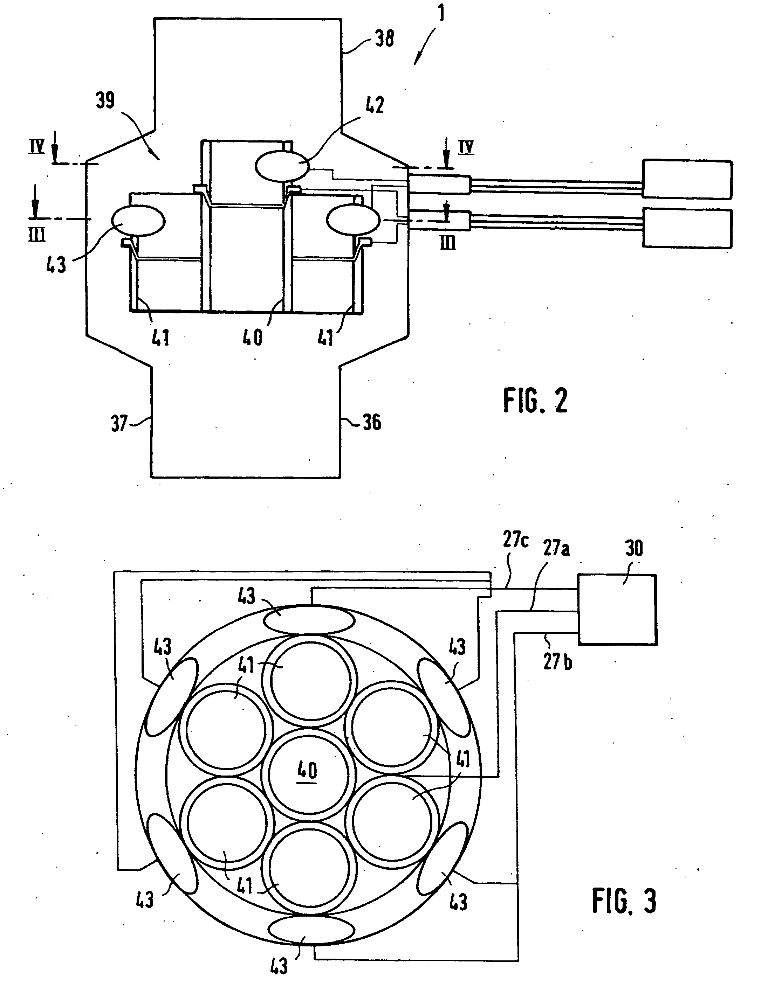

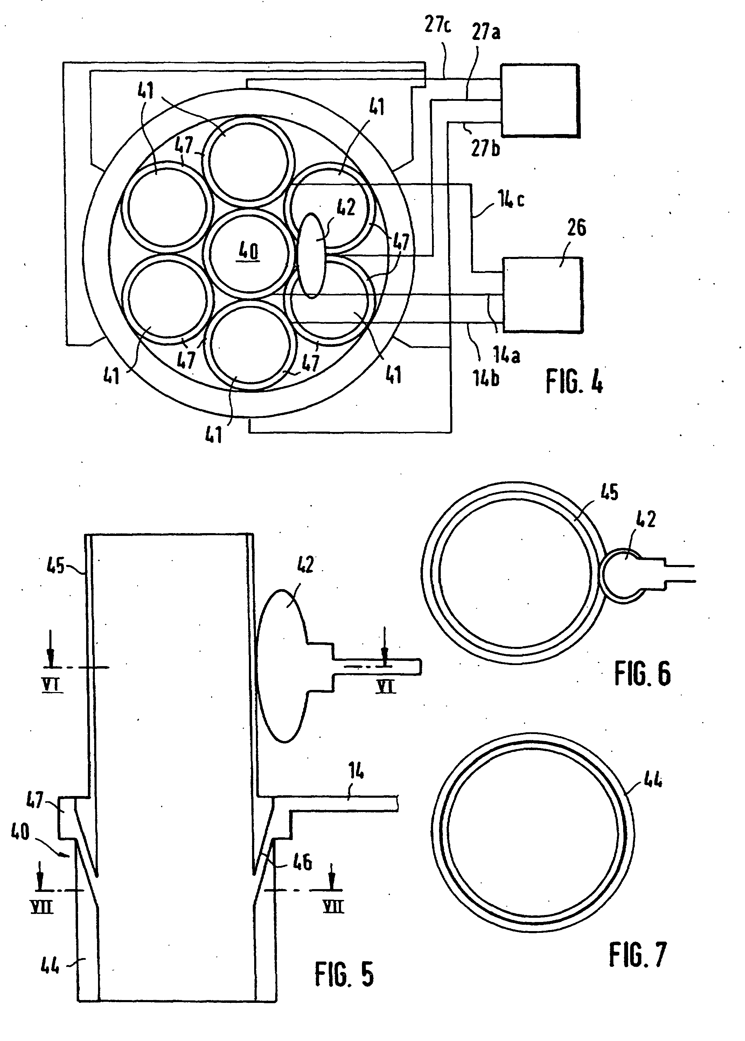

Fig. 2 zeigt in einer vergrößerten Detailansicht den prinzipiellen Aufbau eines Flusseinstellers 1. Dieser besteht aus einem Flusseinstellergehäuse 36, das ein zum Patienten gerichtetes Ende 37 sowie ein zur Umgebung hin offenes Ende 38 aufweist (dieses Ende ist mit den beiden Schläuchen, die die Leitungsschenkel 2 und 3 bilden verbunden). Im Inneren ist eine Düsen- und Blendeneinrichtung 39 vorgesehen, die an ihren Seiten septenartig abgedichtet mit dem Flusseinstellergehäuse 36 verbunden ist, so dass etwaige über das Ende 38 angesaugte Raumluft lediglich durch die Düseneinrichtung hindurchtreten kann. Die Düseneinrichtung selbst besteht aus im gezeigten Ausführungsbeispiel insgesamt sieben Düsen, wobei eine zentrale Düse 40 sowie sechs sie ringförmig umgebende Düsen 41 vorgesehen sind (s. auch Fig. 3 und 4). Jeder Düse 40, 41 ist eine eigene Blende 42 (betreffend die mittlere Düse) bzw. 43 (betreffend die äußeren Düsen) zugeordnet. Wie Fig. 2 zu entnehmen ist, ist die mittlere Düse 40 etwas verlängert und nach oben abstehend gegenüber den anderen Düsen 41 ausgebildet. Hierdurch wird gewährleistet, dass die Blenden 42 problemlos arbeiten kann.

Wie Fig. 5 zeigt besteht jede Düse (in Fig. 5 ist exemplarisch die Düse 40 dargestellt) aus einem unteren, formstabilen Abschnitt 44 und einem an diesen angeordneten elastisch und flexibel verformbaren zweiten Abschnitt 45. An diesem oberen zweiten Abschnitt 45 greift die Blende 42 an. Die Blende 42 kann über eine geeignete Druckgaszuleitung aufgeblasen werden. Wird sie aufgeblasen so drückt sie gegen den flexiblen Abschnitt 45, so dass dieser nach innen gedrückt wird. Hierdurch wird der Querschnitt der Düse verringert und kann bis zum vollständigen Schließen der Düse in jede Stellung variiert werden. Aus den Fig. 3 und 4 ist ersichtlich, dass jeder Blende 40, 41 eine Blende 42, 43 zugeordnet ist. Es ist also möglich, durch Aufblasen der jeweiligen Blenden jeden Düsenquerschnitt verändern zu können.

Wie Fig. 5 ferner zu entnehmen ist, ist am formstabilen Formabschnitt 44 jeder Düse ein umlaufender Injektionskanal 46 vorgesehen. Dieser mündet unter einem spitzen Winkel von vorzugsweise ca. 60° in das Düseninnere. Der Injektionskanal ist ringförmig ausgebildet und ist über einen Verteilerkanal 47 mit der Druckgasleitung 14, die bereits bezüglich Fig. 1 beschrieben wurde, verbunden. Über die Druckgasleitung 14 kann nun ein in dem Sauerstoffmischer 22 aufbereitetes und vom Proportionalventil 24 entsprechend moduliertes sowie von der Verteilereinrichtung 26 verteiltes Gas eingedüst werden. Das trichterförmige Eindüsen bewirkt; dass sich in diesem Bereich eine trichterförmige Turbulenz ausbildet. Hierdurch entsteht ein Überdruck, der dazu führt, dass über das andere Düsenende und damit über das Ende 38 des Flusseinstellers Raumluft nachgesaugt wird. Die Druckdifferenz sowie die Menge der nachgesaugten Raumluft kann durch entsprechende Variation der Blendenstellung und damit des Düsenquerschnitts eingestellt werden.

Wie den Fig. 3 und 4 zu entnehmen ist werden sämtliche Blenden 42, 43 über drei Druckleitungen 27 mit Aufblasgas beaufschlagt. Die Druckleitung 27a läuft zur zentralen Blende 42, die Druckleitung 27b läuft zu den beiden in Fig. 3 rechts unten dargestellten Blenden 43, versorgt also beide. Diese Blenden sind also in ihren Betrieb gekoppelt. Entsprechend läuft die Druckleitung 27c über entsprechende Abzweigungen zu den restlichen peripher verteilten Blenden 43, die ebenfalls gekoppelt sind. Wird also die Druckleitung 27a beaufschlagt, so wird die Blende 42 aufgeblasen, wird die Druckleitung 27b beaufschlagt, so werden die beiden daran gekoppelten Blenden gleichzeitig aufgeblasen, und im Falle der Beaufschlagung der Druckleitung 27c werden sämtliche vier mit dieser gekoppelten Blenden aufgeblasen. Auf diese Weise ist es möglich jede beliebige Blendenanzahl zwischen eins und sieben zu betätigen, je nachdem welche Druckleitung allein oder welche Leitungskombination angesteuert wird. Die Gasverteilung erfolgt über die Verteilereinrichtung 30, die über die Steuerungseinrichtung 31 entsprechend gesteuert wird.

Entsprechend gekoppelt sind auch die jeweiligen Düsen. Die Düse 40 ist über die Druckleitung 14a mit der Verteilereinrichtung verbunden. Die beiden in Fig. 4 rechts unten gezeigten Düsen 41 sind über ihre Verteilerkanäle 47 miteinander gekoppelt. Am Kopplungspunkt mündet die Druckleitung 14b. D.h., diese beiden Düsen werden simultan mit Gas beaufschlagt. Entsprechend sind die verbleibenden vier Düsen 41 ebenfalls im Bereich ihrer Verteilerkanäle 47 miteinander gekoppelt. Lediglich an einer Stelle mündet die Druckleitung 14c. Auch diese vier Düsen werden simultan mit Gas beaufschlagt. Somit ist es auch hier möglich, eine beliebige Anzahl zwischen eins und sieben an Düsen mit Gas oder Gasgemisch zu beaufschlagen.

Die Druckleistung jeder einzelnen Düse 40, 41 ist vom Fluss in den zirkulären Injektionskanälen 46 bzw. vom Druck in den Verteilerkanälen 47 und den Zuleitungen 14 abhängig. Der Druck für die Düsen 40, 41 wird im Proportionalventil 24 erzeugt und mit Hilfe der Verteilereinrichtung 26 über die Leitungen 14 zu den Injektionskanälen verteilt. Mit der Zahl der aktivierten, also mit Gas oder Gasgemisch beaufschlagten Düsen sinkt die Druckleistung des Flusseinstellers. Bei gleichbleibendem Fluss entwickelt der Flussgenerator die höchste Druckleistung mit nur einer beaufschlagten Düse, nämlich hier der separat beaufschlagbaren zentralen Düse 40, alle anderen peripheren Düsen 41 sind deaktiviert. Sie werden also nicht mit Gas beaufschlagt, infolgedessen bilden sich dort keine Druckverhältnisse aus, die entsprechende Turbulenzen erzeugen, die dem Venturieffekt ähnlich sind und ein Ansaugen der Raumluft ermöglichen. Die Zahl der aktivierten Düsen kann während der Inspiration, wenn dem Patienten also Gas zugeführt werden muß, beliebig geändert werden.
Die Blenden 42, 43 komprimieren von außen wie beschrieben die elastischen oberen Enden der jeweiligen Düsen. Der Grad der Komprimierung entspricht wiederum dem Aufblasdruck, wobei auch hier die Aufblasgasverteilung über die Verteilereinheit 30 gesteuert wird. Auf diese Weise wird auch der Widerstand der Exspiration gesteuert und die Menge der eingesaugten Raumluft während der Inspiration begrenzt. Auch die Zahl der aktivierten Blenden kann während der Inspiration oder Exspiration beliebig geändert werden.

Nachfolgend werden die einzelnen Funktionen anhand der Beatmungsbeispiele "Inspiration", "Exspiration", "spontane Atmung", "unterstütze Beatmung", und "kontrollierte Beatmung" näher erläutert.

### 1. "Inspiration"

Während der Inspiration wird über die Zuleitungen 14 zwischen den einzelnen Düsen und deren Injektionskanälen 46 komprimiertes und mit Hilfe des Proportionalventils 24 nach Frequenz, Druck und Zeitverhältnissen moduliertes Gas zugeführt. Aufgrund der durch das Injizieren erzeugten trichterförmigen Turbulenz in der oder den beaufschlagten Düsen entsteht am zum Patienten gewandten Ende 37 des Flusseinstellers ein positiver Druck, der für die Inspiration verwendet wird. Am zur Umgebung offenen Ende 38 des Flusseinstellers 1 entsteht ein Unterdruck, der zum Einsaugen der Raumluft über den inspiratorischen Schenkel 2 verwendet wird. Bei Beatmung mit sauerstoffreichen Gasen wird die Menge der eingesaugten Raumluft mit Hilfe der Blenden 42, 43 durch Variierung des jeweiligen Düsenquerschnitts entsprechend begrenzt.

Die Zahl der aktivierten Düsen 40, 41 wird während der Beatmung entsprechend dem zum Ende der Inspiration sinkenden Fluss und steigendem Lungenwiderstand reduziert. Um volumenkonstante Beatmungsformen zu ermöglichen wird auf diese Weise die Druckleistung des Flusseinstellers gesteigert und der Widerstand der Lunge somit überwunden. Die Steigerung der Druckleistung wird mittels Deaktivierung einiger Düsen erreicht, es wird einigen Düsen also kein Gas mehr injiziert.

Da jede Düse lediglich einen Durchmesser von wenigen mm, insbesondere von ca. 2 mm aufweist, ist das Innenvolumen des Flusseinstellers sehr klein. Da auch die Zuleitungen äußerst dünn sind, da die Gase mit Hochdruck zugeführt werden, ergibt sich insgesamt ein System, das praktisch über keinen Totraum verfügt. Vor jeder Inspiration wird der Flusseinsteller mit Frischgas kurz gespült. Aufgrund dessen und aufgrund des fehlenden Totraums kann er und damit das erfindungsgemäße Beatmungsgerät auch bei Kindern und Säuglingen eingesetzt werden.

### 2. "Exspiration"

Die Exspiration erfolgt passiv. Zur Erzeugung eines positiven exspiratorischen Drucks werden die Blenden 42, 43 synchron oder asynchron, je nach gewünschtem zu erzeugenden Widerstand, aufgeblasen, um die oberen Abschnitte der Düsen 40, 41 teilweise oder vollständig zu komprimieren. Bei Verwendung eines pulsierenden (oszilierenden) exspiratorischen Widerstands werden die Düsen 40, 41 entsprechend aktiviert und mit Gas beaufschlagt. Dies ist abhängig vom gewählten Beatmungsmodus.

### 3. "spontane Atmung"

Der Patient ist in der Lage auch bei ausgeschalteten Zustand des Beatmungsgeräts wie auch im Beatmungsbetrieb über den inspiratorischen und exspiratorischen Schenkel des offenen Beatmungskreises spontan zu atmen.

Das inspiratorische Atemzugvolumen wird mit Hilfe des Flusssensors 6 und 25 gemessen. Das exspiratorische Atemzugsvolumen wird mittels des Flusssensors 8 erfasst. Die Richtungsventile 4 und 5 bestimmen die Zirkulationsrichtung der Atemgase und verfügen über einen minimalen Widerstand. Die Konzentration der exspiratorischen Gase wird mit Hilfe der Sensoren 10 gemessen. Bei Spontanatmung kann das Inspirationsgas mit Sauerstoff angereichert werden. Hierfür wird der Sauerstoff während der Inspiration über die Druckleitung 14 zu den Düsen geführt, die zugeführte Menge wird mit Hilfe des Flusssensors 25 erfasst.
Die inspiratorische Sauerstoffkonzentration wird rechnerisch bestimmt und mit den exspiratorischen Werten des Sensors 10 verglichen. Ausgangspunkt für die rechnerische Ermittlung der Sauerstoffkonzentration sind die Messwerte der Flusssensoren 25, 6 und 8 sowie der die Menge des beigemischten Sauerstoffs angebende Messwert des Sauerstoffmischers 22. Die Beatmungsdrucke werden mit Hilfe des Drucksensors 13 bestimmt. Dieser registriert auch die Atemversuche des Patienten. Sobald ein solcher erfasst wird, wird das Beatmungsgerät entsprechend getriggert. Die Flusstriggerung erfolgt über den Flusssensor 6.

### 4. "Unterstützte Beatmung"

Bei unterstützter Beatmung, die dann einsetzt, wenn der Patient im Stande ist, bereits selbst etwas zu atmen, erfolgt nach Druck- oder Flusstriggerung eine Gaszufuhr zu den Injektionskanälen 46. Dem Patienten wird also zusätzlich zu der von ihm selbst einatembaren Luft noch etwas zusätzliches Gas zugeführt. Während der Inspiration und der Exspiration kann eine hochfrequente Modellierung der Beatmungsdrucke vorgenommen werden, indem das Proportionalventil 24 statt kontinuierlichem Druck hochfrequente Pulsationen generiert. Das Flussmuster kann während der Atmungsphasen über die Steuerungseinrichtung 31 beliebig variiert werden.

### 5. "Kontrollierte Beatmung"

Bei kontrollierter Beatmung ist dem Patienten die Möglichkeit zu spontanen Atemzügen durch das offene System des Beatmungskreises zu jeden beliebigen Zeitpunkt möglich. Denn etwaige Lungentätigkeiten werden sofort über den Drucksensor 13 erfasst, so dass zum einen sofort auf ein aktives Tun des Patienten reagiert werden kann, zum anderen ist ein selbstständiges Inspirieren oder Exspirieren über die offenen Leitungsschenkel 2, 3 jederzeit möglich.

Die vom Flusseinsteller 1 entwickelten Geräusche werden insbesondere im inspiratorischen Schenkel 2 hinreichend gedämpft, so dass zusätzliche Geräuschdämpfer nicht erforderlich sind.

Das erfindungsgemäße Beatmungsgerät bietet eine Reihe von Vorteilen gegenüber dem bekannten Stand der Technik. Da der Flusseinsteller, also der eigentliche Druckerzeuger unmittelbar am Patienten und damit zwischen dem Patienten und dem Beatmungsschläuchen angebracht ist, hat der Totraum und die Trägheit des Gerätes keine negativen Auswirkungen auf die Triggerempfindlichkeit, Hochfrequenzoszillationen werden im Beatmungskreis nicht mehr gedämpft. Beliebige Beatmungsverfahren können mit einem Gerät realisiert werden.

Außerdem ist der Triggerkomfort wesentlich verbessert, da der Flusseinsteller und der Drucksensor 13, (Triggersensor) direkt am Patienten angebracht sind, erfolgt die Triggererkennung und die Unterstützung der Atemversuche des Patienten sehr schnell.

Aufgrund des praktisch nicht gegebenen Totraums ist eine Verwendung des Beatmungsgerät so bei Erwachsenen wie auch bei Neugeborenen möglich.

In den Beatmungsschläuchen, also den Leitungsschenkeln 2, 3 selbst wird kein Druck erzeugt. Sie trennen lediglich den Fluss zwischen der inspiratorischen und der exspiratorischen Sensorik und bilden einen offenen Beatmungskreis, der dem Patienten zu jeder Möglichkeit des maschinellen Atemzyklus die spontane Atmung erlaubt. Bei Bedarf können die Beatmungsschläuche auch weggelassen werden, was einen zusätzlichen Patientenkomfort bietet.

Ferner bietet die Verwendung der Raumluft in dem Multidüsenflusseinsteller die Möglichkeit, bis zu 50 % der komprimierten Luft einzusparen. Auch eine Einsparung an Sauerstoff im beachtlichen Ausmaß ist möglich. Die Steuerungseinrichtung selbst ist derart ausgebildet, dass über sie beliebige Beatmungsverfahren einstellbar und abrufbar sind.

## Patentansprüche

1. Beatmungsgerät, mit einem mit einem Patienten verbindbaren Flusseinsteller (1) für in- und exspiratorisches Gas, der ein zur Umgebung hin offenes distales und ein dem Patienten zugewandtes proximales Ende aufweist, und in dessen Innerem eine Düseneinrichtung vorgesehen ist, der abhängig vom jeweiligen Atemzyklus aus wenigstens einer externen Gasquelle ein Gas oder ein Gasgemisch zuführbar ist, wodurch vor und hinter der Düseneinrichtung unterschiedliche Druckverhältnisse erzeugbar sind, und der eine Blendeneinrichtung (39) zugeordnet ist, und mit einer die Gasoder Gasgemischzufuhr und die Funktion der Blendeneinrichtung (39) steuernden Steuerungseinrichtung (31), **dadurch gekennzeichnet, dass** die Düsen- und die Blendeneinrichtung (39) mehrere parallele, verteilt angeordnete Düsen (40, 41) mit jeweils einer Blende (42, 43) zum Variieren des jeweiligen Düsenquerschnitts aufweist, wobei die Anzahl der mit dem Gas oder Gasgemisch beaufschlagbaren Düsen (40, 41) und der zur Querschnittsvariation zu betätigenden Blenden (42, 43) bedarfsabhängig wählbar ist.

2. Beatmungsgerät nach Anspruch 1, **dadurch gekennzeichnet, dass** eine Blende (42, 43) durch Gaszufuhr balgartig aufblasbar ist, wobei der Düsenquerschnitt abhängig vom Aufblasgrad ist.

3. Beatmungsgerät nach Anspruch 1 oder 2, **dadurch gekennzeichnet, dass** jede Düse (40, 41) separat mit dem Gas oder Gasgemisch beaufschlagbar und/oder jede Blende (42, 43) separat betätigbar ist.

4. Beatmungsgerät nach Anspruch 1 oder 2, **dadurch gekennzeichnet, dass** zumindest ein Teil der Düsen (40, 41) miteinander gekoppelt und gemeinsam mit dem Gas oder Gasgemisch beaufschlagbar ist und/oder dass zumindest ein Teil der Blenden (42, 43) miteinander gekoppelt und gemeinsam betätigbar sind.

5. Beatmungsgerät nach Anspruch 4, **dadurch gekennzeichnet, dass** die Düsen (40, 41) und Blenden (42, 43) derart gekoppelt sind, dass zwischen einer Düse (40, 41) und Blende (42, 43) und der maximalen Anzahl an Düsen (40, 41) und Blenden (42, 43) jede beliebige Düsen- und Blendenanzahl wählbar ist.

6. Beatmungsgerät nach einem der Ansprüche 3 bis 5, **dadurch gekennzeichnet, dass** mindestens fünf, insbesondere mindestens sieben Düsen (40, 41) und Blenden (42, 43) vorgesehen sind.

7. Beatmungsgerät nach Anspruch 5 und 6, **dadurch gekennzeichnet, dass** bei fünf Düsen und Blenden die folgenden Konfiguration gewählt ist:
- je eine separate Düse und Blende,
- zwei Paare mit jeweils zwei gekoppelten Düsen bzw. Blenden, und
dass bei sieben Düsen (40, 41) und Blenden (42, 43) folgende Konfiguration gewählt ist:
- je eine separate Düse (40) und Blende (42),
- zwei Paare mit jeweils zwei gekoppelten Düsen (41) bzw. Blenden (43),
- zwei Paare mit jeweils vier gekoppelten Düsen (41) bzw. Blenden (43), und
dass bei zwölf Düsen und Blenden die folgende Konfiguration gewählt ist:
- je eine separate Düse und Blende,
- zwei Paare mit jeweils zwei gekoppelten Düsen bzw. Blenden,
- zwei Paare mit jeweils vier gekoppelten Düsen bzw. Blenden,
- zwei Paare mit jeweils fünf gekoppelten Düsen bzw. Blenden.

8. Beatmungsgerät nach einem der vorangehenden Ansprüche, **dadurch gekennzeichnet, dass** die Düsen (40, 41) und die Blenden (42, 43) jeweils synchron oder asynchron beaufschlabar bzw. betätigbar sind.

9. Beatmungsgerät nach einem der vorangehenden Ansprüche, **dadurch gekennzeichnet, dass** jeweils eine über die Steuerungseinrichtung (31) steuerbare Verteilereinrichtung (26, 30) zum Zuführen des Gases oder Gasgemischs an die eine oder mehreren Düsen (40, 41) und des Aufblasgases an die eine oder mehreren Blenden (42, 43) vorgesehen ist.

10. Beatmungsgas nach Anspruch 9, **dadurch gekennzeichnet, dass** ein Einstellmittel, insbesondere ein Proportionalventil (24) zum Einstellen der zuzuführenden Gasmengen vorgesehen ist.

11. Beatmungsgerät nach Anspruch 9 oder 10, **dadurch gekennzeichnet, dass** der den Düsen zugeordneten Verteilereinrichtung (26) wenigstens ein Proportionalventil (24) mit zugeordnetem Flusssensor (25) und Druckstabilisator (23) vorgeschaltet ist.

12. Beatmungsgerät nach einem der Ansprüche 9 bis 11, **dadurch gekennzeichnet, dass** der den Blenden zugeordneten Verteilereinrichtung (30) wenigstens ein Druckerzeuger (29) und ein Druckstabilisator (28) vorgeschaltet ist.

13. Beatmungsgerät nach einem der vorangehenden Ansprüche, **dadurch gekennzeichnet, dass** eine zentrale und mittig angeordnete Düse (40) samt Blende (42) vorgesehen ist, die gegenüber den um sie herum verteilten Düsen (41) und Blenden (43) erhöht angeordnet sind.

14. Beatmungsgerät nach einem der vorangehenden Ansprüche, **dadurch gekennzeichnet, dass** die Düsen (40, 41) und Blenden (42, 43) in im Wesentlichen kreisförmiger Konfiguration in einem rohrartigen, vorzugsweise kreisförmigen Flusseinstellergehäuse (36) angeordnet sind.

15. Beatmungsgerät nach einem der Ansprüche 1 bis 14, **dadurch gekennzeichnet, dass** die Düsen und Blenden in einer von einer Kreisform abweichenden Konfiguration in dem rohrförmigen Flusseinstellergehäuse angeordnet sind.

16. Beatmungsgerät nach einem der vorangehenden Ansprüche, **dadurch gekennzeichnet, dass** jede Düse (40, 41) rohrartig ausgebildet ist und einen im Wesentlichen kreisförmigen Querschnitt aufweist.

17. Beatmungsgerät nach einem der Ansprüche 1 bis 15, **dadurch gekennzeichnet, dass** jede Düse rohrartig ausgebildet ist und einen von einer Kreisform abweichenden Querschnitt aufweist.

18. Beatmungsgerät nach einem der vorangehenden Ansprüche, **dadurch gekennzeichnet, dass** jede Düse (40, 41) aus einem ersten formstabilen Abschnitt (44) und einem zweiten elastisch verformbaren Abschnitt (45) besteht, der mittels der Blende (42, 43) zur Querschnittsvariation reversibel zusammendrückbar ist.

19. Beatmungsgerät nach einem der vorangehenden Ansprüche, **dadurch gekennzeichnet, dass** an jeder Düse (40, 41), insbesondere am formstabilen Abschnitt (44) wenigstens ein Injektionskanal (46) vorgesehen ist, über den das Gas oder Gasgemisch in die Düse (40, 41) einbringbar ist.

20. Beatmungsgerät nach Anspruch 19, **dadurch gekennzeichnet, dass** der Injektionskanal (46) unter einem spitzen Winkel bezüglich der Düsenwand und zum unteren, zum Patienten hin gerichteten Ende (37) mündet.

21. Beatmungsgerät nach Anspruch 19 oder 20, **dadurch gekennzeichnet, dass** an jeder Düse (40, 41) ein im Wesentlichen ringförmiger Verteilerkanal (47) vorgesehen ist, über den einzubringendes Gas oder Gasgemisch in den wenigstens einen an der Düse (40, 41) vorgesehenen Injektionskanal (46) einbringbar bzw. zwischen mehreren an der Düse vorgesehenen Injektionskanälen verteilbar ist.

22. Beatmungsgerät nach Anspruch 20, **dadurch gekennzeichnet, dass** gekoppelte Düsen (41) über ihre Verteilerkanäle (47) miteinander gekoppelt sind.

23. Beatmungsgerät nach einem der vorangehenden Ansprüche, **dadurch gekennzeichnet, dass** es als offenes System mit einem zur Umgebung hin offenen inspiratorischen Leitungsschenkel (2) und einem exspiratorischen Leitungsschenkel (3) ausgebildet ist.

24. Beatmungsgerät nach einem der vorangehenden Ansprüche, **dadurch gekennzeichnet, dass** mindestens ein mit der Steuerungseinrichtung (1) kommunizierendes Sensorelement (6, 8, 10, 13, 25, 29) zum Erfassen wenigstens eines beatmungsrelevanten Messwertes des In- und/oder Exspirationsgases, in dessen Abhängigkeit die Steuerung der Düsen (40, 41) und Blenden (42, 43) erfolgt, vorgesehen ist.

## Claims

1. Respirator, having a flow adjuster (1) which is connectable to the patient for inhaled and exhaled gas, which has a distal end open to the environment and a proximal end associated with the patient, and in whose interior a nozzle device is provided, to which a gas or gas mixture is suppliable from at least one external gas source depending on the particular breathing cycle, whereby different pressure conditions may be produced upstream and downstream of the nozzle device, and to which a diaphragm device (39) is allocated, and having a control device (31) for controlling the gas or gas mixture supply and the function of the diaphragm device (39), **characterised in that** the nozzle and diaphragm device (39) has plural parallel, distributed nozzles (40, 41) each with a diaphragm (42, 43) for varying the respective nozzle cross-section, wherein the number of nozzles (40, 41) that may be acted on by the gas or gas mixture and of diaphragms (42, 43) that may be actuated for varying the cross-section is selectable according to need.

2. Respirator according to claim 1, **characterised in that** a diaphragm (42, 43) may be inflated like a bellows by the gas supply, the nozzle cross-section depending on the degree of inflation.

3. Respirator according to claim 1 or 2, **characterised in that** each nozzle (40, 41) may be acted on separately with the gas or gas mixture and/or each diaphragm (42, 43) may be actuated separately.

4. Respirator according to claim 1 or 2, **characterised in that** at least some of the nozzles (40, 41) are coupled together and may be acted on in common by the gas or gas mixture and/or **in that** at least some of the diaphragms (42, 43) are coupled together and may be actuated in common.

5. Respirator according to claim 4, **characterised in that** the nozzles (40, 41) and diaphragms (42, 43) are so coupled together that between one nozzle (40, 41) and one diaphragm (42, 43) and the maximum number of nozzles (40, 41) and diaphragms (42, 43), any desired number of nozzles and diaphragms may be selected.

6. Respirator according to one of claims 3 to 5, **characterised in that** at least five, in particular at least seven nozzles (40, 41) and diaphragms (42, 43) are provided.

7. Respirator according to claim 5 and 6, **characterised in that** in the case of five nozzles and diaphragms the following configuration is selected:
- one separate nozzle and diaphragm in each case,
- two pairs each with two coupled nozzles and diaphragms respectively,
and **in that** in the case of seven nozzles (40, 41) and diaphragms (42, 43) the following configuration is selected:
- one separate nozzle (40) and diaphragm (42) in each case,
- two pairs each of two coupled nozzles (41) and diaphragms (43) respectively,
- two pairs each of four coupled nozzles (41) and diaphragms (43) respectively,
and **in that** in the case of twelve nozzles and diaphragms, the following configuration is selected:
- one separate nozzle and diaphragm in each case,
- two pairs each of two coupled nozzles and diaphragms respectively,
- two pairs each of four coupled nozzles and diaphragms respectively,
- two pairs each of five coupled nozzles and diaphragms respectively.

8. Respirator according to one of the preceding claims, **characterised in that** the nozzles (40, 41) and the diaphragms (42, 43) are respectively capable of being acted on or are actuatable synchronously or asynchronously.

9. Respirator according to one of the preceding claims, **characterised in that** a respective distributor (26, 30) is provided which is controllable via a control device (31) for supplying the gas or gas mixture to the one or more nozzles (40, 41) and for supplying the inflating gas to the one or more diaphragms (42, 43).

10. Respirator according to claim 9, **characterised in that** an adjusting means, in particular a proportional valve (24) is provided for setting the quantities of gas to be supplied.

11. Respirator according to claim 9 or 10, **characterised in that** at least one proportional valve (24) with associated flow sensor (25) and pressure stabiliser (23) is connected upstream of the distributor (26) allocated to the nozzles.

12. Respirator according to one of claims 9 to 11, **characterised in that** at least one pressure generator (29) and a pressure stabiliser (28) are connected upstream of the distributor (30) allocated to the diaphragms.

13. Respirator according to one of the preceding claims, **characterised in that** a concentric and centrally disposed nozzle (40) is provided together with a diaphragm (42), which are arranged proud of the nozzles (41) and diaphragms (43) distributed around the same.

14. Respirator according to one of the preceding claims, **characterised in that** the nozzles (40, 41) and diaphragms (42, 43) are arranged in a substantially circular configuration in a tubular, preferably circular flow adjuster housing (36).

15. Respirator according to one of claims 1 to 14, **characterised in that** the nozzles and diaphragms are arranged in a configuration in the tubular flow adjuster housing that differs from a circular form.

16. Respirator according to one of the preceding claims, **characterised in that** each nozzle (41, 41) is tubular in form and has a substantially circular cross-section.

17. Respirator according to one of claims 1 to 15, **characterised in that** each nozzle is tubular in form and has a cross-section which is other than circular.

18. Respirator according to one of the preceding claims, **characterised in that** each nozzle (40, 41) consists of a first, rigid section (44) and a second, resiliently deformable section (45), which is reversibly compressible by means of the diaphragm (42, 43) in order to vary the cross-section.

19. Respirator according to one of the preceding claims, **characterised in that** at least one injection channel (46) is provided on each nozzle (40, 41), in particular on the rigid section (44), via which channel the gas or gas mixture may be introduced into the nozzle (40, 41).

20. Respirator according to claim 19, **characterised in that** the injection channel (46) opens at an acute angle to the nozzle wall and to the lower end (37) oriented towards the patient.

21. Respirator according to claim 19 or 20, **characterised in that** on each nozzle (40, 41) a substantially annular distributor channel (47) is provided, via which gas or gas mixture may be introduced into the at least one injection channel (46) provided on the nozzle (40, 41) or is distributable between plural injection channels provided on the nozzle.

22. Respirator according to claim 20, **characterised in that** coupled nozzles (41) are coupled together via their distributor channels (47).

23. Respirator according to one of the preceding claims, **characterised in that** it takes the form of an open system with an inhalation conducting arm (2) open to the environment and an exhalation conducting arm (3).

24. Respirator according to one of the preceding claims, **characterised in that** at least one sensor element (6, 8, 10, 13, 25, 29) communicating with the control device (1) is provided for detecting at least one respiration-relevant reading of the inhaled and/or exhaled gas, according to which the nozzles (40, 41) and diaphragms (42, 43) are controlled.

## Revendications

1. Respirateur muni d'un régulateur de flux (1) du gaz d'inspiration et d'expiration, qui est destiné à être relié à un patient et qui comporte une extrémité distale ouverte vers l'environnement et une extrémité proximale orientée vers le patient, et à l'intérieur duquel est prévu un dispositif d'injection, vers lequel est acheminé, en fonction du cycle de respiration, un gaz ou un mélange gazeux à partir d'au moins une source de gaz externe, différentes conditions de pression pouvant ainsi être produites en amont et en aval du dispositif d'injection, et auquel est associé un dispositif d'obturation (39), et muni d'un dispositif de commande (31) qui commande l'admission du gaz ou mélange gazeux et le fonctionnement du dispositif d'obturation (39), **caractérisé en ce que** le dispositif d'injection et dispositif d'obturation (39) comporte plusieurs buses (40, 41) parallèles, agencées selon une répartition donnée, et munies chacune d'un obturateur (42, 43) destiné à varier la section de la buse concernée, le nombre de buses (40, 41) à alimenter en gaz ou mélange gazeux et le nombre d'obturateurs (42, 43) à actionner pour faire varier la section pouvant être choisis en fonction des besoins.

2. Respirateur selon la revendication 1, **caractérisé en ce qu'**un obturateur (42, 43) peut être gonflé à la manière d'un soufflet par l'admission du gaz, la section de la buse étant fonction du niveau de gonflage.

3. Respirateur selon la revendication 1 ou 2, **caractérisé en ce que** chaque buse (40, 41) peut être alimentée séparément en gaz ou mélange gazeux et/ou chaque obturateur (42, 43) peut être actionné séparément.

4. Respirateur selon la revendication 1 ou 2, **caractérisé en ce qu'**au moins une partie des buses (40, 41) sont couplées entre elles et peuvent être alimentées conjointement en gaz ou mélange gazeux et/ou **en ce qu'**au moins une partie des obturateurs (42, 43) sont couplés entre eux et peuvent être alimentés conjointement.

5. Respirateur selon la revendication 4, **caractérisé en ce que** les buses (40, 41) et les obturateurs (42, 43) sont couplés de telle sorte que l'on peut choisir un nombre quelconque de buses et obturateurs entre une buse (40, 41) et un obturateur (42, 43) et le nombre maximum de buses (40, 41) et obturateurs (42, 43).

6. Respirateur selon l'une quelconque des revendications 3 à 5, **caractérisé en ce qu'**il est prévu au moins cinq, en particulier au moins sept buses (40, 41) et obturateurs (42, 43).

7. Respirateur selon les revendications 5 et 6, **caractérisé en ce que**, en présence de cinq buses et obturateurs, on choisit la configuration suivante :
- respectivement une buse séparée et un obturateur séparé,
- deux paires contenant chacune respectivement deux buses couplées et obturateurs couplés,
et **en ce que**, en présence de sept buses (40, 41) et obturateurs (42, 43), on choisit la configuration suivante :
- respectivement une buse (40) séparée et un obturateur (42) séparé,
- deux paires contenant chacune respectivement deux buses couplées (41) et obturateurs couplés (43),
- deux paires contenant chacune respectivement quatre buses couplées (41) et obturateurs couplés (43),
et **en ce que**, en présence de douze buses et obturateurs, on choisit la configuration suivante :
- respectivement une buse séparée et un obturateur séparé,
- deux paires contenant chacune respectivement deux buses couplées et obturateurs couplés,
- deux paires contenant chacune respectivement quatre buses couplées et obturateurs couplés,
- deux paires contenant chacune respectivement cinq buses couplées et obturateurs couplés.

8. Respirateur selon l'une quelconque des revendications précédentes, **caractérisé en ce que** chacune des buses (40, 41) et chacun des obturateurs (42, 43) peuvent être alimentés et actionnés de manière synchrone et asynchrone.

9. Respirateur selon l'une quelconque des revendications précédentes, **caractérisé en ce que** respectivement un distributeur (26, 30), destiné à être commandé par le dispositif de commande (31), est prévu pour acheminer le gaz ou mélange gazeux vers l'une ou plusieurs buses (40, 41) et le gaz de gonflage vers l'un ou plusieurs obturateurs (42, 43).

10. Respirateur selon la revendication 9, **caractérisé en ce qu'**il est prévu un moyen de réglage, plus particulièrement un clapet régulateur proportionnel (24), avec un capteur de flux (25) et un stabilisateur de pression (23) associés.

11. Respirateur selon la revendication 9 ou 10, **caractérisé en ce qu'**au moins une vanne proportionnelle (24) avec capteur de flux (25) attribué et un stabilisateur de pression (23) sont branchés en amont de l'appareil répartiteur (26) attribué aux buses.

12. Respirateur selon l'une quelconque des revendications 9 à 11, **caractérisé en ce qu'**au moins un générateur de pression (29) et un stabilisateur de pression (28) sont montés en amont du distributeur (30) associé aux obturateurs.

13. Respirateur selon l'une quelconque des revendications précédentes, **caractérisé en ce qu'**il est prévu une buse (40) centrale et agencée au milieu, y compris l'obturateur (42), lesquels sont surélevés par rapport aux buses (41) et obturateurs (43) répartis autour d'eux.

14. Respirateur selon l'une quelconque des revendications précédentes, **caractérisé en ce que** les buses (40, 41) et les obturateurs (42, 43) sont agencés selon une configuration sensiblement circulaire dans un boîtier du régulateur de flux (36) tubulaire, de préférence circulaire.

15. Respirateur selon l'une quelconque des rever dications 1 à 14, **caractérisé en ce que** les buses et les obturateurs sont agencés selon une configuration s'écartant de la forme circulaire dans un boîtier du régulateur de flux tubulaire.

16. Respirateur selon l'une quelconque des revendications précédentes, **caractérisé en ce que** chaque buse (40, 41) est conçue en forme de tube et possède une section sensiblement ronde.

17. Respirateur selon l'une quelconque des revendications 1 à 15, **caractérisé en ce que** chaque buse est conçue en forme cie tube et possède une section s'écartant de la forme ronde.

18. Respirateur selon l'une quelconque des revendications précédentes, **caractérisé en ce que** chaque buse (40, 41) est formée par un premier tronçon (44) de forme stable et un deuxième tronçon (45) à déformation élastique qui, pour la variation de la section, peut être comprimé de manière réversible par l'obturateur (42, 43).

19. Respirateur selon l'une quelconque des revendications précédentes, **caractérisé en ce que** sur chaque buse (40, 41), en particulier sur le tronçon (44) de forme stable, il est prévu au moins un canal d'injection (46) par l'intermédiaire duquel le gaz ou le mélange gazeux peuvent être introduits dans la buse (40, 41).

20. Respirateur selon la revendication 19, **caractérisé en ce que** le canal d'injection (46) débouche en formant un angle aigu par rapport à la paroi de la buse et vers l'extrémité inférieure (37) orientée vers le patient.

21. Respirateur selon la revendication 19 ou 20, **caractérisé en ce qu'**il est prévu sur chaque buse (40, 41) un canal de distribution (47) sensiblement en forme d'anneau, par l'intermédiaire duquel le gaz ou mélange gazeux à introduire peut être introduit dans au moins un canal d'injection (46), prévu sur la buse (40, 41), ou peut être réparti entre plusieurs canaux d'injection prévus sur la buse.

22. Respirateur selon la revendication 20, **caractérisé en ce que** des buses (41) couplées sont couplées entre elles par l'intermédiaire de leurs canaux de distribution (47).

23. Respirateur selon l'une quelconque des revendications précédentes, **caractérisé en ce qu'**il est conçu sous forme de système ouvert avec une branche de guidage inspiratoire (2), ouverte vers l'environnement, et une branche de guidage expiratoire (3).

24. Respirateur selon l'une quelconque des revendications précédentes, **caractérisé en ce qu'**il est prévu au moins un élément capteur (6, 8, 10, 13, 25, 29), communiquant avec le dispositif de commande (31) et destiné à déterminer au moins une valeur de mesure significative de la respiration pour le gaz d'inspiration et/ou le gaz d'expiration, en fonction duquel est effectuée la commande des buses (40, 41) et des obturateurs (42, 43).
